# EUROPEAN PATENT APPLICATION

(11) **EP 4 270 297 A1**
(43) Date of publication of application: **01.11.2023**
(21) Application number: 21910271.2
(22) Date of filing: 07.12.2021
(51) Int. Cl.: G06T 1/00, A61B 5/1172, G06T 7/00

(54) **INFORMATION PROCESSING DEVICE, METHOD, AND PROGRAM**

(30) Priority: 22.12.2020 JP 2020212935
(71) Applicant: Sony Group Corporation, Tokyo 108-0075 (JP)
(72) Inventor: ENOKIDO, Toshio, Tokyo 108-0075 (JP)
(74) Representative: MFG Patentanwälte Meyer-Wildhagen Meggle-Freund Gerhard PartG mbB
(86) International application number: PCT/JP2021/044809
(87) International publication number: WO 2022/138121

(57) **Abstract**

To suppress a decrease in authentication accuracy. An information processing apparatus according to an embodiment includes an acquisition unit (21) that acquires image information including biometric information of a target, a condition recognition unit (22) that determines a condition of the target based on the image information, a projection unit (31) that projects light on the target based on a result of the determination by the condition recognition unit, and an authentication unit (32) that performs authentication based on the image information of the target acquired by the acquisition unit in a state where the light is projected.

## Description

### Field

The present disclosure relates to an information processing apparatus, an information processing method, and a program.

### Background

With the development of information processing technology, biometric authentication that is personal authentication using biometric information being biologically unique information has been performed. As the biometric authentication, for example, fingerprint authentication is known.

In a general fingerprint authentication device, authentication is performed using the shape of a fingerprint ridge and bifurcation points and ending points of the ridges called minutiae, as features. In addition, a fingerprint authentication device mounted on a smartphone or the like is divided into a registration phase for registration of a user's fingerprint for permission of login, and a matching phase for matching of the fingerprint upon actual user's login.

In order to succeed in matching in the matching phase while ensuring high security, it is necessary to always match the user's fingerprint obtained upon registration in the registration phase and the normal user's fingerprint, even in different environments upon matching.

### Citation List

### Patent Literature

Patent Literature 1: JP 2017-196319 A

### Summary

### Technical Problem

However, in conventional biometric authentication devices, when it is difficult to acquire accurate biometric information, for example, when a finger has a bad condition, such as exfoliation of finger cuticle, including rough finger and dry finger, there is a problem that accurate features cannot be extracted from the acquired biometric information, reducing authentication accuracy.

The present disclosure has been made in view of such situations, and an object of the present disclosure is to provide an information processing apparatus, an information processing method, and a program that are configured to suppress a decrease in authentication accuracy.

### Solution to Problem

An information processing apparatus according to the present disclosure includes: an acquisition unit that acquires image information including biometric information of a target; a condition recognition unit that determines a condition of the target based on the image information; a projection unit that projects light on the target based on a result of the determination by the condition recognition unit; and an authentication unit that performs authentication based on the image information of the target acquired by the acquisition unit in a state where the light is projected.

### Brief Description of Drawings

FIG. 1 is a schematic diagram illustrating an exemplary schematic configuration of an information processing apparatus according to a first embodiment.
FIG. 2 is an external perspective view of the information processing apparatus according to the first embodiment.
FIG. 3 is a flowchart illustrating exemplary operations according to the first embodiment.
FIG. 4 is an explanatory diagram of an operation of a projection unit according to a second embodiment.
FIG. 5 is a schematic diagram illustrating an exemplary schematic configuration of the projection unit according to the second embodiment.
FIG. 6 is a flowchart illustrating exemplary operations according to the second embodiment.
FIG. 7 is a flowchart illustrating exemplary operations according to a first modification of the second embodiment.
FIG. 8 is a flowchart illustrating exemplary operations according to a second modification of the second embodiment.
FIG. 9 is a schematic diagram illustrating an exemplary schematic configuration of an information processing apparatus according to a third embodiment.
FIG. 10 is a flowchart illustrating exemplary operations according to the third embodiment.
FIG. 11 is a flowchart illustrating exemplary operations according to a fourth embodiment.
FIG. 12 is an explanatory diagram of a cosmetic cream product data group according to the fourth embodiment.
FIG. 13 is an explanatory diagram of recommended cosmetic cream information and exemplary presentation according to the fourth embodiment.
FIG. 14 is a flowchart illustrating a modification according to the fourth embodiment.
FIG. 15 is a schematic diagram of a computer constituting the information processing apparatus according to embodiments and modifications thereof.

### Description of Embodiments

Embodiments of the present disclosure will be described in detail below with reference to the drawings. Note that in the following embodiments, the same portions are denoted by the same reference numerals, and a repetitive description thereof will be omitted.

Furthermore, the present disclosure will be described in the order of items shown below.
1. First Embodiment
   1.1 Exemplary configuration
   1.2 Exemplary operations according to first embodiment
   1.3 Conclusion
2. Second Embodiment
   2.1 Exemplary schematic configuration of projection unit
   2.2 Exemplary operations according to second embodiment
   2.3 Conclusion
   2.4 Modifications of second embodiment
   2.4.1 First modification
   2.4.2 Second modification
   2.4.3 Conclusion
3. Third Embodiment
   3.1 Exemplary configuration
   3.2 Exemplary operations according to third embodiment
   3.3 Conclusion
4. Fourth Embodiment
   4.1 Exemplary operations according to fourth embodiment
   4.2 Modification of fourth embodiment
   4.3 Conclusion
5. Hardware configuration

### 1. First Embodiment

A first embodiment will be described first in detail with reference to the drawings.

### 1.1 Exemplary configuration

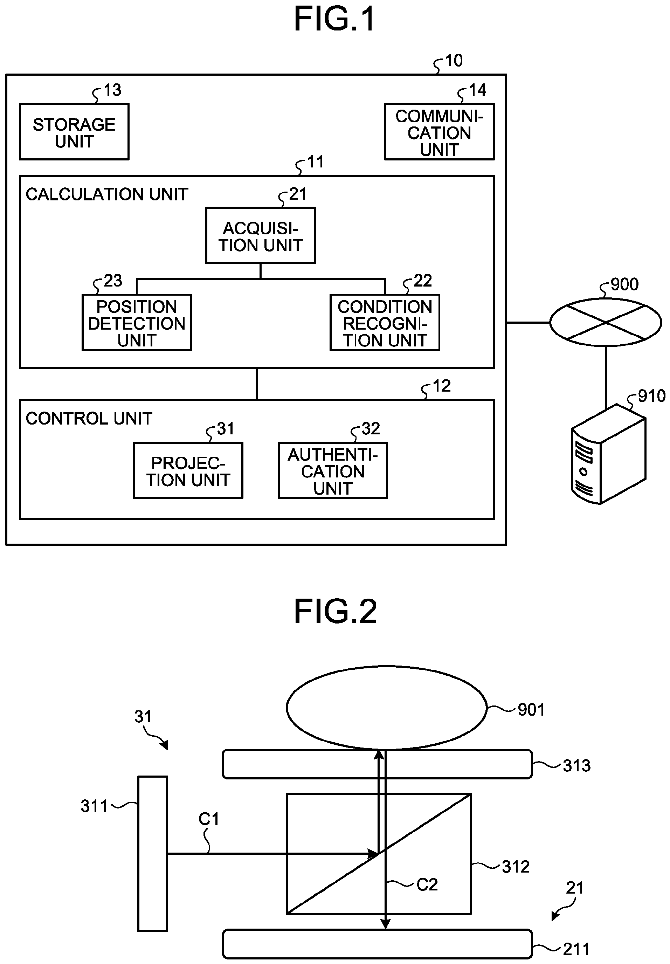

FIG. 1 is a schematic diagram illustrating an exemplary schematic configuration of an information processing apparatus according to the first embodiment. As illustrated in FIG. 1, an information processing apparatus 10 according to the present embodiment includes a calculation unit 11, a control unit 12, a storage unit 13, and a communication unit 14.

### <Calculation unit 11>

The calculation unit 11 calculates various data necessary for operations performed by the information processing apparatus 10. The calculation unit 11 includes an acquisition unit 21, a condition recognition unit 22, and a position detection unit 23.

### (Acquisition unit 21)

The acquisition unit 21 acquires image information about a sensing target by using a sensor. As long as the sensor can acquire the image information, any of various sensors may be employed, and the sensor may be, for example, an image sensor. Furthermore, an infrared sensor, a near infrared sensor, a time of flight (TOF) sensor, or the like may be used. Furthermore, the sensor may include an optical system sensor including a micro lens array (MLA), an event-based vision sensor (EVS), or the like. Note that the sensor is not limited to the above example, and may be various sensors that can acquire the image information about the sensing target. Furthermore, the image information is not limited to two-dimensional image information, and may be one-dimensional image information, three-dimensional image information, or higher Furthermore, the acquisition unit 21 may acquire the image information about the sensing target acquired by a sensor in an information processing apparatus 910 arranged outside the information processing apparatus 10, through a network 900.

Here, examples of the sensing target for sensing by the information processing apparatus 10 include surfaces or the like of parts of a human body or biological body including, for example, surfaces of parts of a hand and foot, such as fingers (thumb (first finger), index finger (second finger), middle finger, ring finger (fourth finger), and little finger), a palm (thenar, hypothenar, carpus), and the back of the hand, a surface of a face, and a surface of an arm. As described above, a sensing area is not limited, and may be human skin or the like. In the following description, the sensing target that is a finger will be exemplified. The acquisition unit 21 acquires, for example, fingerprint image information as biometric information for authentication. However, the biometric information (In this description, also referred to as authentication information) for authentication is not limited to a fingerprint image, and various biometric information such as palm print information, vein information of a hand, a finger, and the like, iris information, and eye vein information may be used, and the authentication information acquired by the acquisition unit 21 may use one piece or two or more pieces of the information.

### (Condition recognition unit 22)

For example, the condition recognition unit 22 determines whether a finger condition sensed is sufficiently good to perform accurate fingerprint authentication, on the basis of the fingerprint image information acquired by the acquisition unit 21. For example, image processing may be used for a determination method. In addition, the finger condition may be determined using machine learning. For a learning method in this configuration, for example, a neural network learning or deep learning can be used.

In the present embodiment, the finger condition can include a dry finger whose surface has cuticle keratinized due to drying, and a normal finger from which normal fingerprint image information is acquired without keratinization. Furthermore, condition information can include information indicating a specific condition such as whether the sensing target has a dry condition or a normal condition, information obtained by scoring the dry condition (information obtained by scoring a degree of dryness), or the like. However, the finger condition is not limited thereto, and may include a condition in which the finger is contaminated with oil, ink, or the like, a condition in which the finger has a cut, or the like, and these conditions may be managed by the condition information called abnormal condition different from the dry condition.

### (Position detection unit 23)

For example, the position detection unit 23 determines whether which range of the finger is detected by the sensor, on the basis of the fingerprint image information (hereinafter, also referred to as fingerprint image) acquired by the acquisition unit 21, and detects a relative position of the finger with respect to the sensor, on the basis of a result of the determination. For example, the position detection unit 23 generates ideal image information about the fingerprint (hereinafter, referred to as ideal fingerprint image) including ideal authentication information for each user on the basis of user's fingerprint information in registration in the registration phase, determines whether the sensor detects which range of the finger on the basis of a positional relationship between the ideal fingerprint image and an actual user's fingerprint image, and detects whether the finger is displaced with respect to the sensor in which direction and how much on the basis of a result of the determination. As a detection method, for example, the finger condition may be determined by using machine learning. For a learning method in this configuration, for example, a neural network learning or deep learning can be used. However, the present disclosure is not limited thereto, and the position detection unit 23 may detect whether the sensor detects which range of the finger, for example, on the basis of various information indicating the features of the finger such as the shape of the ball of the finger or the contour of the finger or on the basis of the degree of contact with the sensor detected using a proximity sensor or the like. Note that the relative position of the finger with respect to the sensor is not limited to a relative position in a horizontal direction, and may be a relative position in a vertical direction or a relative position in both the horizontal direction and the vertical direction.

Furthermore, for example, when the condition recognition unit 22 determines that the user's finger is not the normal finger, the position detection unit 23 recognizes whether a dry portion having the dry condition or abnormal portion having the abnormal condition, including a position of cuticle on a finger surface or a position contaminated with oil or ink, is located at which position, from the fingerprint image. Note that, in the present description, the "portion" or the "position" may be an area having a range.

### <Control unit 12>

The control unit 12 controls the operations of the respective units included in the information processing apparatus 10. Furthermore, the control unit 12 includes a projection unit 31 and an authentication unit 32.

### (Projection unit 31)

The projection unit 31 is, for example, a projector, and projects light, an image, or the like on the sensing target. For example, the projection unit 31 projects the ideal fingerprint image on the finger, for each user. However, the projection unit 31 may project, on the finger, an image generated on the basis of a previous fingerprint used for matching in fingerprint authentication by the user or a fingerprint registered in the registration phase in the past, instead of the ideal fingerprint image.

As illustrated in FIG. 2, the projection unit 31 includes a light source 311 and a projection optical system 312.

The light source 311 is, for example, a point light source, a line light source, or a surface light source, such as a light emitting diode (LED), an organic LED (OLED), or a vertical cavity surface emitting laser (VCSEL). The light source 311 may be a monochromatic light source or a polychromatic light source (including a white light source). Furthermore, in a case where the monochromatic light source is used, the light source 311 may be a green light source, an infrared light source, or the like.

The projection optical system 312 includes, for example, at least one optical element such as a half mirror or a prism. The projection optical system 312 is arranged at a position where an optical axis C1 of the light source 311 and an optical axis C2 of a sensor 211 in the acquisition unit 21 intersect, and makes the optical axis C1 of light, an image, or the like emitted from the light source 311 substantially coincide with the optical axis C2 of the sensor 211. Therefore, the light or image projected from the light source 311 is projected on the surface of a finger 901 positioned on the optical axis C2 of the sensor 211.

Note that a light receiving surface of the sensor 211 on which the projection optical system 312 is arranged may be covered with a transparent cover 313 that transmits at least light emitted from the light source 311 to prevent a finger or the like from making direct contact with the projection optical system 312 or the sensor 211. In that configuration, the light or image projected from the light source 311 may be projected on the surface of the finger 901 making contact with or approaching the transparent cover 313.

In addition, for example, the projection unit 31 may project the ideal fingerprint image on the dry portion or the abnormal portion detected by the position detection unit 23, on the basis of the relative position of the finger with respect to the sensor 211 detected by the position detection unit 23. Furthermore, the ideal fingerprint image may be projected using a geometric correction function such as keystone correction so that ridges in the ideal fingerprint image and ridges on the finger surface are matched. This configuration makes it possible to complement a lost portion of the fingerprint image by the projected ideal fingerprint image, increasing authentication accuracy for the dry finger or the like.

Furthermore, for example, the projection unit 31 may project a specific figure on the finger. The specific figure may be a basic figure such as a square shape or a lattice shape. The position detection unit 23 may detect distortion of the figure projected on the finger surface to detect the center position of the finger.

Specifically, for example, the position detection unit 23 may detect magnification information about the figure at each position on the basis of distortion at each position in the figure projected on the finger surface to detect the center position of the finger by using a distribution of the detected magnification information. At that time, for example, the position detection unit 23 may calculate a distance between the finger and the sensor 211 by using a difference in magnification of each position, as the magnification information to detect unevenness of the finger from calculation results. A position where the distance between the finger and the sensor 211 is the shortest is the most protruding position on the finger surface, and thus, this position can be detected as the center position of the finger. Furthermore, the curvature of the finger may be calculated from a distortion rate of any projected figure by using an identical magnification as the magnification information to detect the most protruding position as the center position. However, the present disclosure is not limited thereto, and may use a difference in resolution between the respective positions, as resolution information to detect a position having the highest resolution, that is, a position where the finger and the sensor 211 are closest to each other, detecting this position as the center position.

Furthermore, the projection unit 31 may project, on the finger surface, complementary information (e.g., image) for complementing the lost portion of the fingerprint image, which is generated by using machine learning. For example, the complementary information may be generated by a trained model with the fingerprint image acquired by the acquisition unit 21 as an input. For a learning method in this configuration, for example, a neural network learning or deep learning can be used. Furthermore, the complementary information may be the ideal fingerprint image. Furthermore, the generation of the complementary information may be performed by the projection unit 31, or may be performed by an arithmetic unit, which is not illustrated, or the like included in the control unit 12.

Furthermore, the acquisition unit 21 may include a proximity sensor or the like, and the projection unit 31 may start projection at timing at which approaching of the finger is detected using the proximity sensor or the like.

### (Authentication unit 32)

The authentication unit 32 performs authentication processing for the user, for example, by using the image information acquired by the acquisition unit 21. For example, the authentication unit 32 matches the image information acquired by the acquisition unit 21 and the fingerprint information registered in the registration phase to perform user authentication. Note that the fingerprint image registered in the registration phase may be acquired by the acquisition unit 21 in the information processing apparatus 10, or may be acquired by using another information processing apparatus or a fingerprint image acquisition apparatus. For this user authentication, for example, pattern matching for comparing feature points between images, machine learning, or the like may be used. However, the present disclosure is not limited to these methods.

### <Storage unit 13>

The storage unit 13 stores, for example, information obtained by the calculation unit 11 and the control unit 12. The stored information includes the image information acquired by the acquisition unit 21, the condition information acquired by the condition recognition unit 22, position information detected by the position detection unit 23, the ideal fingerprint image generated by the position detection unit 23, the user's fingerprint image acquired by the authentication unit 32 or in another information processing apparatus, and the like. However, the present disclosure is not limited thereto. Note that the storage unit 13 may be arranged in the information processing apparatus 10 or may be arranged, for example, on the network 900 outside the information processing apparatus 10.

### <Communication unit 14>

The communication unit 14 is, for example, a communication interface for communication with another device via the network 900 in a wireless and/or wired manner, and, for example, transmits/receives information input to/output from the calculation unit 11, the control unit 12, and the storage unit 13 to/from another information processing apparatus.

### 1.2 Exemplary operations according to first embodiment

Next, exemplary operations according to the first embodiment will be described in detail.

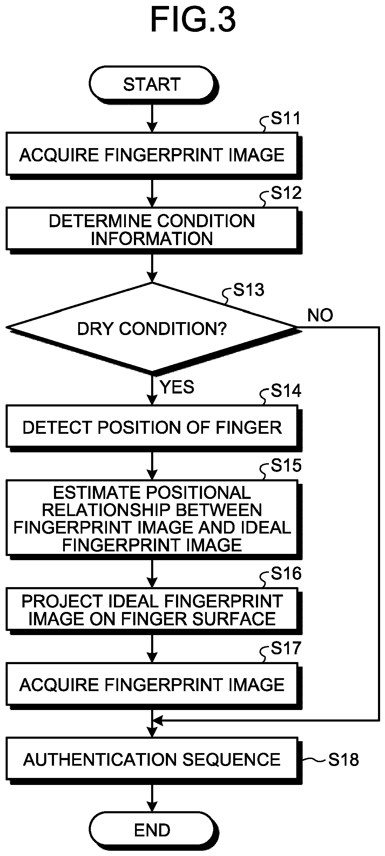

FIG. 3 is a flowchart illustrating the exemplary operations according to the first embodiment.

In this operation, first, the acquisition unit 21 acquires the user's fingerprint image acquired by the sensor 211 (Step S11). Note that, in a case where the acquisition unit 21 includes the sensor 211, the acquisition unit 21 drives the sensor 211 to acquire the user's fingerprint image. The acquired user's fingerprint image is input to the condition recognition unit 22 in the calculation unit 11. Furthermore, in a case where the sensor 211 is arranged outside the information processing apparatus 10, the acquisition unit 21 may acquire the fingerprint image acquired by the sensor 211 via the network 900.

Next, the condition recognition unit 22 determines finger condition information on the basis of the input user's fingerprint image (Step S12). In this determination processing, as described above, condition determination by image processing, condition determination using machine learning, and the like may be performed.

When a result of the determination in Step S12 indicates that the finger has no dry condition, that is, the finger has the normal condition (Step S13; NO), the user's fingerprint image acquired in Step S11 is input to the authentication unit 32, and the authentication unit 32 performs the authentication processing based on the user's fingerprint image (Step S18). Thereafter, the present operations end.

On the other hand, when a result of the determination by the condition recognition unit 22 indicates the dry condition (Step S13; YES), the user's fingerprint image acquired in Step S11 is input to the position detection unit 23, and the position detection unit 23 detects the position of the finger (Step S14). At that time, the position detection unit 23 may detect the dry portion on the finger surface, in addition to the position of the finger.

Next, on the basis of the position of the finger detected in Step S14 and the user's ideal fingerprint image read from the storage unit 13, the position detection unit 23 estimates the positional relationship between the fingerprint image acquired in Step S11 and the ideal fingerprint image (Step S15). Note that, for this estimation, for example, pattern matching for matching feature points (center position, ridge pattern, etc.) between the fingerprint image and the ideal fingerprint image, estimation of the positional relationship by using machine learning, or the like may be used.

When the positional relationship between the fingerprint image and the ideal fingerprint image is estimated in this manner, the control unit 12 drives the projection unit 31 on the basis of this positional relationship to project the ideal fingerprint image aligned with the user's fingerprint, on the finger surface (Step S16). Note that the control unit 12 may drive the projection unit 31 before Step S15 to project the ideal fingerprint image for a reference position, on the user's finger surface. In this state, the control unit 12 may directly estimate the positional relationship between the actual position of the finger and the projection position of the ideal fingerprint image, on the basis of a displacement between the actual positions of the ridges or the like on the finger surface and the positions of the ridges or the like in the ideal fingerprint image projected on the finger surface, in the image acquired by the acquisition unit 21, and project, on the finger surface, the ideal fingerprint image aligned with the user's fingerprint according to the estimated positional relationship.

In addition, in Step S14, when the position detection unit 23 has detected not only the position of the finger but also the dry portion on the finger surface, the control unit 12 may drive the projection unit 31 so that the ideal fingerprint image is partially projected on the dry portion, in Step S16. In addition, the image projected in Step S16 is not limited to the ideal fingerprint image, and may be a light pattern that selectively illuminates a normal portion having the normal condition to lower the illuminance at the dry portion. Therefore, it is possible to reduce overexposure of the image at the dry portion in the fingerprint image due to reflection at the dry portion, thus suppressing deterioration in image quality of the fingerprint image.

In addition, the light source 311 projecting the light pattern may have OLED so that the OLED serves as liquid crystal and the sensor 211 is arranged under the liquid crystal. In other words, the present disclosure is not limited to the configuration in which the light source 311 is arranged beside the sensor 211 as illustrated in FIG. 2.

Projecting the ideal fingerprint image by the projection unit 31 complements the dry portion on the user's finger surface with the fingerprint information. Therefore, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18). Thereafter, the present operations end.

### 1.3 Conclusion

As described above, according to the present embodiment, the ideal fingerprint image is projected according to the dry condition of the finger, whereby the loss of the fingerprint is complemented, and it is possible to suppress the decrease in the authentication accuracy.

Note that, in the present embodiment, light emitted by the light source 311 is not limited to visible light, and may employ, for example, infrared light (including near-infrared light and far-infrared light) or the like. Furthermore, the light emitted from the light source 311 is not limited to monochromatic light, and may employ multicolor light (including light with a multi-wavelength spectrum, white light with a broad wavelength spectrum, etc.).

### 2. Second Embodiment

Next, a second embodiment will be described in detail with reference to the drawings. Note that, in the following description, for configurations, operations, and effects similar to those of the embodiments described above, the similar configurations, operations, and effects of the embodiments described above are cited, and the redundant descriptions thereof will be omitted.

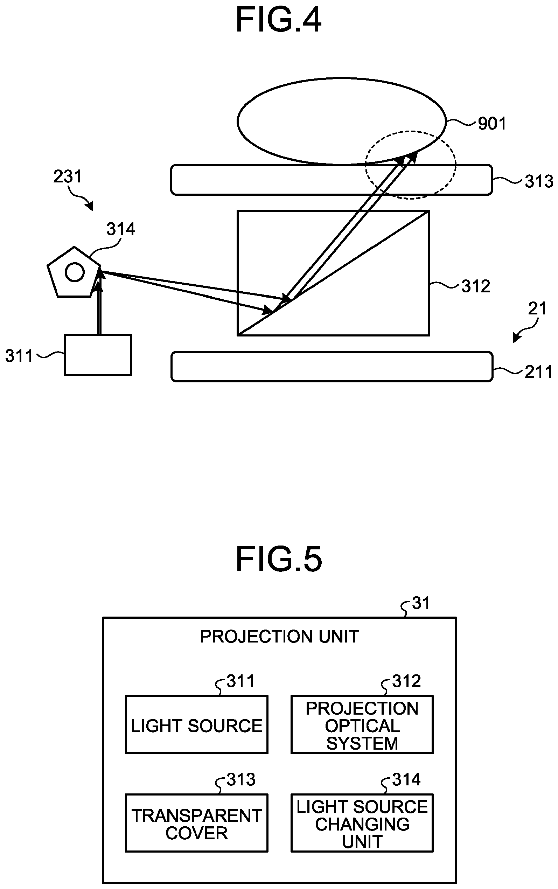

An information processing apparatus according to the present embodiment may be similar to, for example, the information processing apparatus 10 described with reference to FIG. 1 in the first embodiment. However, in the second embodiment, the projection unit 31 described with reference to FIG. 2 in the first embodiment is replaced with a projection unit 231 illustrated in FIG. 4.

### 2.1 Exemplary schematic configuration of projection unit

FIG. 5 is a schematic diagram illustrating an exemplary schematic configuration of the projection unit according to the second embodiment. As illustrated in FIG. 4, the projection unit 231 further includes, for example, a light source changing unit 314 in addition to the light source 311 and the projection optical system 312 described with reference to FIG. 2 in the first embodiment.

The light source changing unit 314 is an optical system for controlling the optical axis of light emitted from the light source 311. The light source changing unit 314 includes, for example, an optical scanning device such as a galvanometer mirror, polygon mirror, or optical micro electro mechanical systems (MEMS), and controls the optical axis of the light emitted from the light source 311. For example, angled light is projected on the finger surface to form a shadow in conformity with the fingerprint ridges, on the finger surface, whereby it is possible to create the complementary information that complements information lost due to drying or contamination, from shadow information generated. This configuration makes it possible to improve accuracy in matching the fingerprint of the dry finger or the like. Note that the light source changing unit 314 is not limited to a reflective optical system as described above, and may include a transmissive optical system such as a prism, or a combination thereof. Furthermore, the angle of the light source 311 itself may be changed to change the projection angle of light. In that configuration, the light source changing unit 314 may be an attitude control mechanism that controls the attitude of the light source 311.

### 2.2 Exemplary operations according to second embodiment

Next, exemplary operations according to the second embodiment will be described in detail.

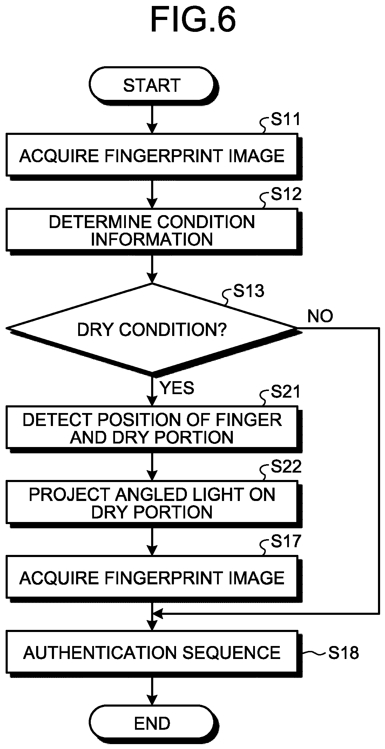

FIG. 6 is a flowchart illustrating exemplary operations according to the second embodiment. Note that, in the description of the exemplary operations according to the present embodiment, for operations similar to those of the exemplary operations of the first embodiment described with reference to FIG. 3, the similar operations of the first embodiment are cited, and detailed descriptions thereof will be omitted.

In the present operations, first, operations similar to the operations shown in Steps S11 to S13 of FIG. 3 are performed in the first embodiment to determine the finger condition information on the basis of the fingerprint image acquired by the acquisition unit 21. When the finger has the normal condition (Step S13; NO), the authentication processing based on the fingerprint image acquired in Step S11 is performed (Step S18).

On the other hand, when a result of the determination in Step S12 indicates that the finger has the dry condition (Step S13; YES), the user's fingerprint image acquired in Step S11 is input to the position detection unit 23, and the position detection unit 23 detects the position of the finger and the dry portion on the finger surface (Step S21).

Next, the control unit 12 drives the projection unit 231 on the basis of the position of the finger detected in Step S21 and the dry portion on the finger surface to project angled light on the dry portion on the finger surface (Step S22).

Projection of the angled light on the dry portion on the finger surface by the projection unit 231 forms the shadow in conformity with the fingerprint ridges, on the finger surface, thereby complementing the dry portion on the user's finger surface. Therefore, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18). Thereafter, the present operations end.

### 2.3 Conclusion

As described above, according to the present embodiment, projection of the angled light on the dry portion on the finger surface forms the shadow in conformity with the fingerprint ridges, on the finger surface, whereby a clearer fingerprint image can be acquired. Therefore, it is possible to suppress the decrease in the authentication accuracy.

Note that the other configurations, operations, and effects may be similar to those of the embodiments described above, and detailed description thereof will be omitted here.

### 2.4 Modifications of second embodiment

Next, examples according to modifications of the second embodiment described above will be described.

### 2.4.1 First modification

In the second embodiment, a mode has been exemplified in which the angled light is projected on the dry portion on the finger surface to acquire the clearer fingerprint image with reduced influence of the dry condition, but the present disclosure is not limited thereto, and, for example, a focus of a lens of the sensor 211 in the acquisition unit 21 may be changed using an actuator or the like so as not to detect the finger surface having the dry condition.

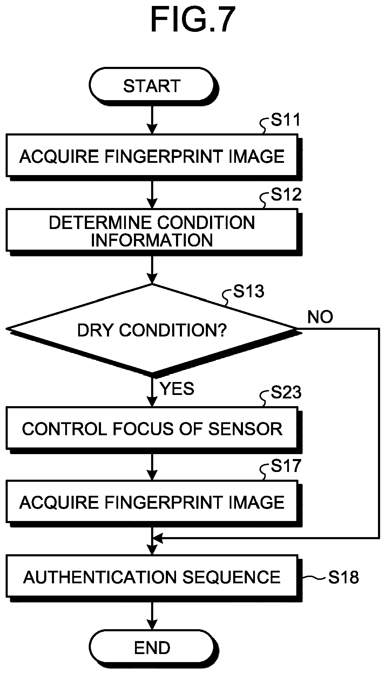

FIG. 7 is a flowchart illustrating exemplary operations according to a first modification of the second embodiment. Note that, in the description of the exemplary operations according to the present modification, for operations similar to those of the exemplary operations of the second embodiment described with reference to FIG. 6, the similar operations of the first embodiment are cited, and detailed descriptions thereof will be omitted.

In the present operations, first, operations similar to the operations shown in Steps S11 to S13 of FIG. 6 are performed in the second embodiment to determine the finger condition information on the basis of the fingerprint image acquired by the acquisition unit 21. When the finger has the normal condition (Step S13; NO), the authentication processing based on the fingerprint image acquired in Step S11 is performed (Step S18).

On the other hand, when a result of the determination in Step S12 indicates that the finger has the dry condition (Step S13; YES), for example, a position of the lens of the sensor 211 in the acquisition unit 21 is moved by using an actuator, not illustrated, or the like, and the focus of the sensor 211 is controlled to a position displaced from the user's finger surface (Step S23). At that time, displacement of the focus of the sensor 211 from the finger surface to an extent that the image of the ridges on the finger surface does not disappear makes it possible to reduce the influence of the dry portion in the fingerprint image while leaving the image of the ridges in the fingerprint image acquired by the sensor 211.

Thereafter, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18). Thereafter, the present operations end.

### 2.4.2 Second modification

In a second modification, a description is made of preventing detection of the finger surface having the dry condition by controlling the wavelength of light emitted from the light source 311, instead of controlling the focus of the sensor 211 in the first modification.

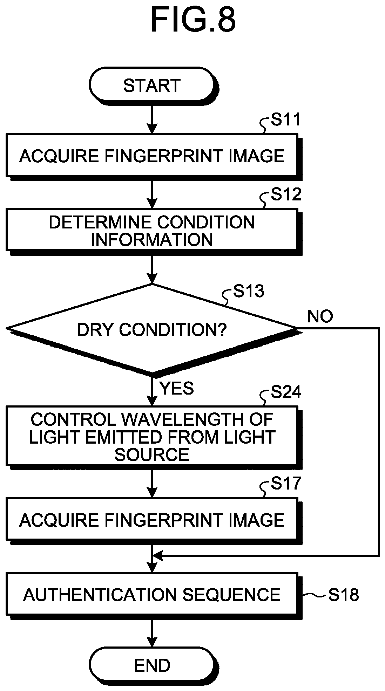

FIG. 8 is a flowchart illustrating exemplary operations according to the second modification of the second embodiment. Note that, in the description of the exemplary operations according to the present modification, for operations similar to those of the exemplary operations of the second embodiment described with reference to FIG. 6, the similar operations of the first embodiment are cited, and detailed descriptions thereof will be omitted.

In the present operations, first, operations similar to the operations shown in Steps S11 to S13 of FIG. 6 are performed in the second embodiment to determine the finger condition information on the basis of the fingerprint image acquired by the acquisition unit 21. When the finger has the normal condition (Step S13; NO), the authentication processing based on the fingerprint image acquired in Step S11 is performed (Step S18).

On the other hand, when a result of the determination in Step S12 indicates that the finger has the dry condition (Step S13; YES), for example, the light source 311 in the projection unit 231 is controlled to control the wavelength of light projected on the user's finger surface (Step S24). For example, in a case where the light source 311 includes a plurality of light sources that outputs light of different wavelengths, the control unit 12 may switch the light source 311 to be driven in Step S24 to control the wavelength of the light projected on the user's finger surface.

Thereafter, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18). Thereafter, the present operations end.

Note that, in the present operations, the light source 311 driven in normal imaging (e.g., imaging in Step S11) may be a visible light source such as a green light source, and the light source 311 driven by switching in Step S24 may be, for example, a light source emitting light outside the visible light range such as an infrared light source. For example, when the light projected on the user's finger surface is infrared light or near-infrared light, reflection of the light on the finger surface is reduced, whereby it is possible to blur the image of the dry portion in the acquired fingerprint image. Therefore, it is possible to acquire an image more suitable for authentication, thereby suppressing the decrease in the authentication accuracy.

### 2.4.3 Conclusion

As described above, according to the present modification, controlling the focus of the lens of the sensor 211 in the acquisition unit 21 or controlling the wavelength of light emitted makes it possible to reduce the influence of the dry portion while detecting the ridges on the finger surface, whereby it is possible to acquire the fingerprint image more preferable for the fingerprint authentication. Therefore, it is possible to suppress the decrease in the authentication accuracy.

### 3. Third Embodiment

First, a third embodiment will be described in detail with reference to the drawings. Note that, in the following description, for configurations, operations, and effects similar to those of the embodiments described above, the similar configurations, operations, and effects of the embodiments described above are cited, and the redundant descriptions thereof will be omitted.

### 3.1 Exemplary configuration

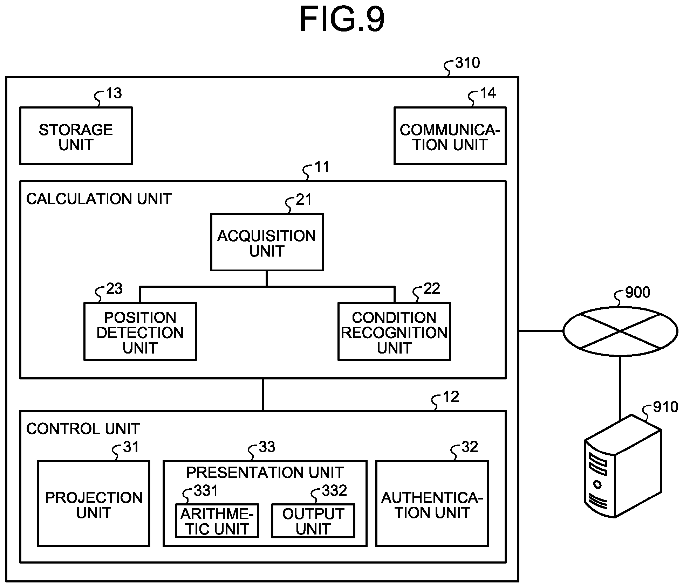

FIG. 9 is a schematic diagram illustrating an exemplary schematic configuration of an information processing apparatus according to the third embodiment. As illustrated in FIG. 9, in an information processing apparatus 310 according to the present embodiment, the control unit 12 further includes a presentation unit 33, in a configuration similar to that of the information processing apparatus 10 described with reference to FIG. 1 in the first embodiment.

The presentation unit 33 includes, for example, an arithmetic unit 331 and an output unit 332, and presents a method for improving the dry condition to the user by using an image, sound, a message, or the like, on the basis of the position of the finger or the dry portion detected by the position detection unit 23. For example, a direction or position for moving the position of the finger are presented, at a position having fewer dry portions, on the basis of the dry portion. In this configuration, presentation for moving the position of the finger may be performed by grasping the dry portions of the entire finger, or the presentation for moving the position of the finger may be performed by estimating an area with fewer dry portions (area having fewer portions where the authentication information is lost) by using machine learning, and any method can be used. In addition, the portions where the authentication information is lost may be grasped as the area. In addition, when, for example, it is difficult to perform the fingerprint authentication processing due to a large number of dry portions, the presentation unit 33 may guide the user to try another authentication method. Note that various information to be presented to the user may be stored in the storage unit 13 or may be managed in an information processing apparatus 910 connected via the network 900.

### 3.2 Exemplary operations according to third embodiment

Next, exemplary operations according to the third embodiment will be described in detail.

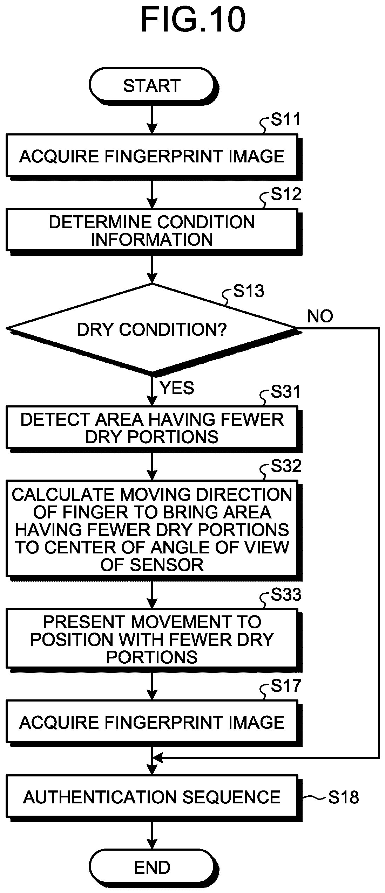

FIG. 10 is a flowchart illustrating exemplary operations according to the third embodiment. Note that, in the description of the exemplary operations according to the present embodiment, for operations similar to those of the exemplary operations of the first embodiment described with reference to FIG. 3 or FIG. 6, the similar operations of the first embodiment are cited, and detailed descriptions thereof will be omitted.

In the present operations, first, operations similar to the operations shown in Steps S11 to S13 of FIG. 6 are performed in the second embodiment to determine the finger condition information on the basis of the fingerprint image acquired by the acquisition unit 21. When the finger has the normal condition (Step S13; NO), the authentication processing based on the fingerprint image acquired in Step S11 is performed (Step S18).

On the other hand, when a result of the determination in Step S12 indicates that the finger has the dry condition (Step S13; YES), the user's fingerprint image acquired in Step S11 is input to the position detection unit 23, and the position detection unit 23 detects an area on the finger surface having fewer dry portions (Step S31). In Step S31, for example, the area having fewer dry portions may be detected, on the basis of a distribution of the dry portions detected by a method similar to the method of detecting the dry portions shown in Step S21 of FIG. 6.

The area having fewer dry portions detected in this manner is input to the arithmetic unit 331 in the presentation unit 33. The arithmetic unit 331 calculates a moving direction (direction and distance, in some cases) of the finger to bring the area on the finger surface having fewer dry portions to the center of an angle of view of the sensor 211 of the acquisition unit 21 (Step S32). Information about the calculated moving direction and the like is input to the output unit 332. Then, the output unit 332 presents the movement of the finger to the user, on the basis of the input information about the moving direction and the like of the finger (Step S33).

Note that a method of presenting the moving direction or the like of the finger to the user by the output unit 332 in Step S33 is not limited, and an arrow indicating a direction in which the finger is to be moved may be projected on the transparent cover 313 by using the projection unit 31 or an arrow along which the finger is to be moved may be displayed by using a display unit (not illustrated) of the information processing apparatus 310. In addition, the direction along which the finger is to be moved may be indicated by voice. Furthermore, the moving direction and the like of the finger for bringing the area on the finger surface having fewer dry portions to the center of the angle of view of the sensor are not limited to the methods illustrated in Steps S31 to S32, and the moving direction and the like of the finger may be estimated by, for example, machine learning with the fingerprint image input in Step S11 as an input. In this configuration, in Step S33, the output unit 332 presents the movement of the finger, to the user, on the basis of the estimated information about the moving direction and the like of the finger.

When the user moves the finger in accordance with the presentation by the presentation unit 33 upon the presentation in Step S33, an area on the finger surface closer to the normal condition is within the angle of view of the sensor 211 of the acquisition unit 21. Therefore, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18). Thereafter, the present operations end.

### 3.3 Conclusion

As described above, according to the present embodiment, the area on the finger surface having fewer dry portions moves to the center, and therefore, the clearer fingerprint image can be obtained. Therefore, it is possible to suppress the decrease in the authentication accuracy.

Note that the other configurations, operations, and effects may be similar to those of the embodiments described above, and detailed description thereof will be omitted here.

### 4. Fourth Embodiment

Next, a fourth embodiment will be described in detail with reference to the drawings. Note that, in the following description, for configurations, operations, and effects similar to those of the embodiments described above, the similar configurations, operations, and effects of the embodiments described above are cited, and the redundant descriptions thereof will be omitted.

In the exemplary operations according to the third embodiment described above, the presentation unit 33 gives a presentation for the user to bring the area on the finger surface having fewer dry portions to the center of the angle of view of the sensor. However, instead of presentation of the moving direction or the like of the finger to the user, presentation of information for improving the dry condition, that is, information for encouraging the user to reduce an area on the finger surface where the fingerprint information is lost may be given. This information may include recommendation information for recommending, for the user, a method, product, or the like for improving the condition of the finger surface that is the target. For example, when a product for improving the condition of the finger surface is recommended to the user, the recommendation information may include information indicating the product (also referred to as recommended product information). Furthermore, the product recommended to the user at that time may be a cosmetic product or the like, and more specifically, may be a moisturizer such as a milky lotion or cosmetic cream (also referred to as cosmetic cream product). In the following description, recommendation of the cosmetic cream to the user, as a product for improving the condition of the finger surface will be exemplified. Therefore, in the following description, the recommended product information is referred to as recommended cosmetic cream information.

Furthermore, the recommended cosmetic cream information presented to the user to encourage the user to apply the cosmetic cream may include information about ingredients effective for improving the finger condition, or the like, instead of or in addition to information (product name etc.) for identifying a cosmetic cream more suitable for the finger condition.

Note that the information processing apparatus according to the present embodiment may have a configuration similar to that of the information processing apparatus 310 described with reference to FIG. 9 in the third embodiment, for example. However, in the present embodiment, the information processing apparatus 310 performs operations exemplified below.

### 4.1 Exemplary operations according to fourth embodiment

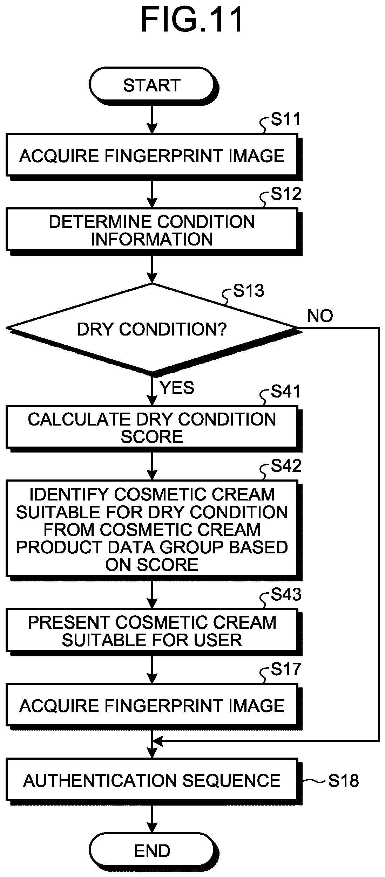

FIG. 11 is a flowchart illustrating exemplary operations according to the fourth embodiment. Note that, in the description of the exemplary operations according to the present embodiment, for operations similar to those of the exemplary operations described with reference to FIGS. 3, 6, 10, and the like, the similar operations described with reference to FIGS. 3, 6, 10, and the like are cited, and detailed descriptions thereof will be omitted.

In the present operations, first, operations similar to the operations shown in Steps S11 to S13 of FIG. 8 are performed in the third embodiment to determine the finger condition information on the basis of the fingerprint image acquired by the acquisition unit 21. When the finger has the normal condition (Step S13; NO), the authentication processing based on the fingerprint image acquired in Step S11 is performed (Step S18).

On the other hand, when a result of the determination in Step S12 indicates that the finger has the dry condition (Step S13; YES), the condition recognition unit 22 calculates a dry condition score included in the condition information (Step S41), in the third embodiment. The dry condition score is information obtained by scoring the degree of dryness, and, for example, machine learning using a trained model trained by using an image of the finger surface and a value obtained by scoring the dry condition of the finger surface as training data may be used for calculation of the score.

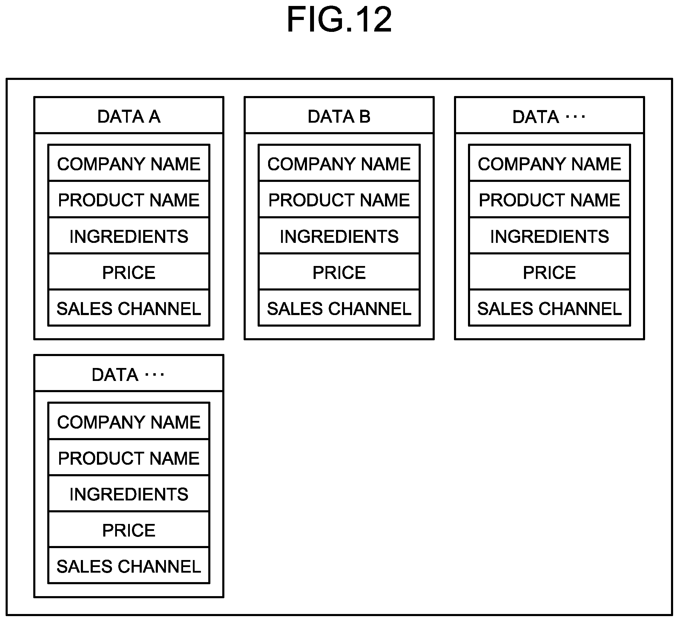

Next, the arithmetic unit 331 identifies a cosmetic cream suitable for the dry condition of the user's finger from, for example, a product data group (In the present example, a cosmetic cream product data group) as exemplified in FIG. 12, on the basis of the dry condition score calculated in Step S41 (Step S42). The cosmetic cream product data group is a data group that stores data such as a company name, product name, ingredients, price, and sales channel. The cosmetic cream product data group may be a data table such as a lookup table, or may be data having a data structure such as a database. Furthermore, the cosmetic cream product data group may be stored, for example, in the storage unit 13 in the information processing apparatus 310, or may be stored in another information processing apparatus 910 (may be a database) connected to the information processing apparatus 310 via the network 900. Note that the present disclosure is not limited to the cosmetic cream product, and a product that may improve the dry condition, such as a skin lotion or milky lotion, may be presented to the user, instead of or together with the cosmetic cream product.

In this way, when the cosmetic cream product to be presented to the user is identified, the identified recommended cosmetic cream information is presented to the user (Step S43). Note that, here, the recommended cosmetic cream information may only be presented, or application of the cosmetic cream may be encouraged. In addition, a product similar to the identified recommended cosmetic cream information may be identified from among the cosmetic cream product data group. Furthermore, for example, a data group of user's cosmetic cream products may be created. The data group of user's cosmetic cream products may be stored, for example, in the storage unit 13 in the information processing apparatus 310, or may be stored in another information processing apparatus 910 (may be a database) connected to the information processing apparatus 310 via the network 900. The recommended cosmetic cream information may be presented from among the user's cosmetic cream products by using this data group.

Encouraging the user to apply an optimal cosmetic cream to improve the dry condition of the finger surface by the presentation unit 33 facilitates detection of the fingerprint ridges on the finger surface, and the fingerprint information on the user's finger surface is acquired. Therefore, the control unit 12 acquires the user's fingerprint image again via the acquisition unit 21 (Step S17). The fingerprint image acquired again is input to the authentication unit 32, and the authentication processing based on the user's fingerprint image is performed in the authentication unit 32 (Step S18).

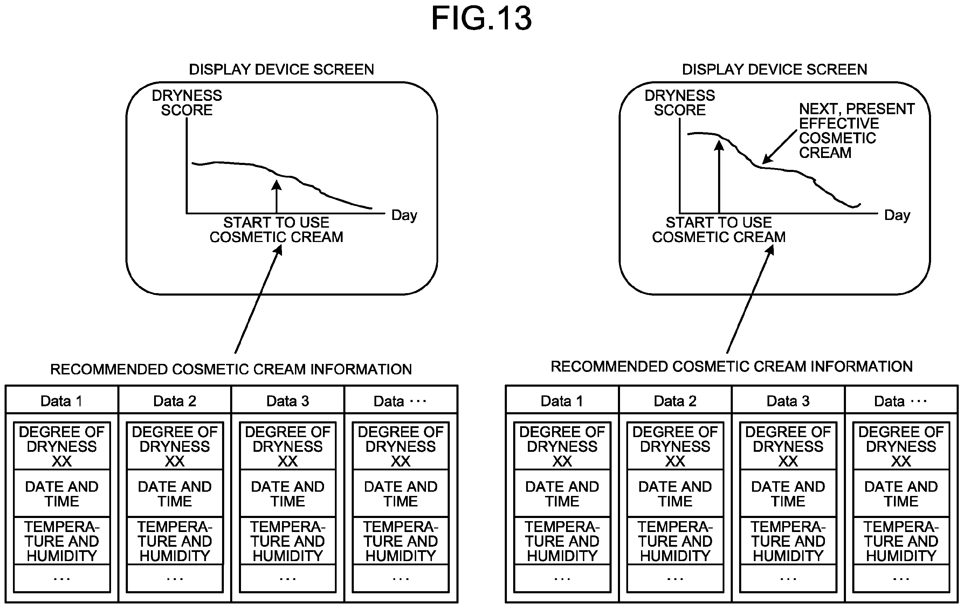

Note that as illustrated in FIG. 13, the storage unit 13 may accumulate the recommended cosmetic cream information in log, including date, time, temperature, and humidity upon identification of the cosmetic cream suitable for the dry condition of the user's finger in Step S42, the dry condition score calculated in Step S41, and information for identifying the identified cosmetic cream. This log may be stored, for example, in the storage unit 13 in the information processing apparatus 310, or may be stored in another information processing apparatus 910 (may be a database) connected to the information processing apparatus 310 via the network 900.

The arithmetic unit 331 may calculate, as time-series information, whether how the dryness score changes before and after the start of use of the cosmetic cream, on the basis of the recommended cosmetic cream information that includes date, time, temperature, and humidity upon identification of the cosmetic cream and further includes the dry condition score calculated in Step S41. As illustrated in FIG. 13, the output unit 332 may graphically display temporal change in the dryness score calculated by the arithmetic unit 331.

In addition, when there is little change in a state having a low dry condition score (reduced dryness) and the dry condition is not improved, the following recommended cosmetic cream information may be presented. The timing of presentation is not limited, and for example, an elapsed time from the presentation of the previous recommended cosmetic cream information may be measured and presented at specific timing, such as timing at which it is estimated that the cosmetic cream has been consumed or timing after a predetermined duration in which the dry condition is not improved continues.

### 4.2 Modification of fourth embodiment

Next, an example according to a modification of the fourth embodiment described above will be described.

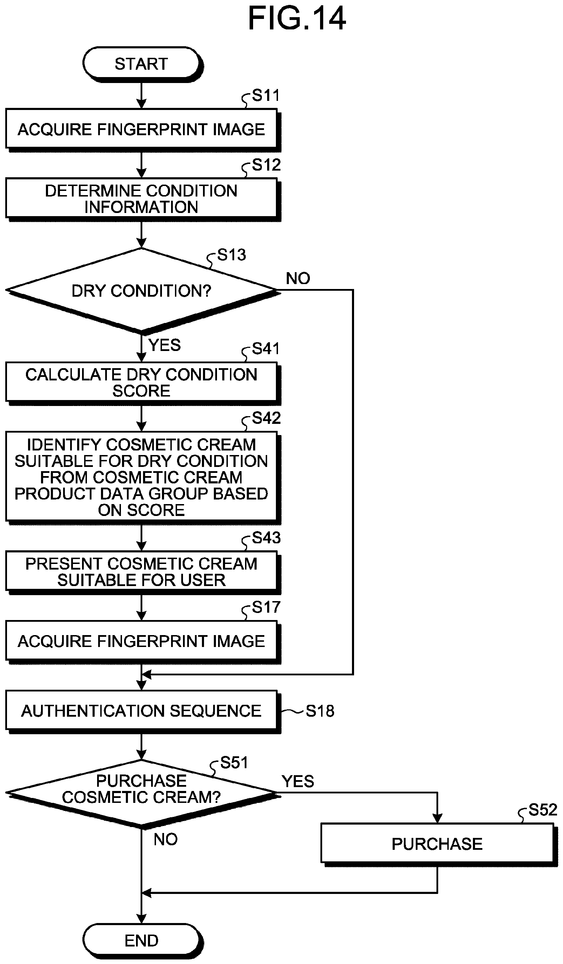

FIG. 14 is a flowchart illustrating a modification according to the fourth embodiment. Note that, in the description of the modification according to the present embodiment, for operations similar to the exemplary operations described above with reference to FIGS. 3, 6, 10, 11, and the like, the similar operations described above are cited, and detailed descriptions thereof will be omitted.

As illustrated in FIG. 14, in the operations according to the present modification, to the similar operations to the operations described in the fourth embodiment with reference to FIG. 11, an operation for the user to purchase a recommended cosmetic cream product (and/or another product) is added, after the user authentication in Step S18,

Specifically, when the user authentication is completed in Step S18, for example, the output unit 332 confirms that the user intends to purchase the presented cosmetic cream product (Step S51). At that time, the recommended cosmetic cream information presented in Step S43 may be presented to the user again, or the recommended cosmetic cream information presented in Step S43 may be continuously displayed until Step S51. Furthermore, user's purchase intention may be confirmed, for example, by presenting a button of purchase/non-purchase. The operation for confirming the user's purchase intention is not limited to this operation, and the user's purchase intention may be confirmed on the basis of a response such as a voice input or response message to presentation such as an image, voice, or message presented to the user. When the purchase intention cannot be confirmed (Step S51; NO), the present operations end.

On the other hand, when the purchase intention can be confirmed (Step S51; YES) the arithmetic unit 331 cooperates with, for example, an e-commerce site, and makes a purchase of the identified cosmetic cream product and payment, on the basis of the recommended cosmetic cream information given by the arithmetic unit 331 (Step S52). Thereafter, the present operations end. Note that the method of purchasing the cosmetic cream product is not limited thereto, and for example, purchase by the user himself/herself may be supported by guiding the user to a cosmetic cream product buying site. In addition, in the purchase of and payment for the cosmetic cream product, for example, the payment may be made using a result of the authentication processing in Step S18, or the payment may be made upon success in the authentication processing performed again before Step S52.

### 4.3 Conclusion

As described above, according to the present embodiment, the user is encouraged to apply the optimal cosmetic cream to improve the dry condition of the finger surface, and whereby detection of the fingerprint ridges on the finger surface is facilitated. Therefore, it is possible to acquire a clearer fingerprint image, thereby suppressing the decrease in the authentication accuracy.

Note that in the present embodiment, encouraging removal of the dry condition of the finger by using the cosmetic cream or another product has been exemplified, but the method for removing the dry condition of the finger is not limited thereto. For example, the following configuration may be employed to eliminate the dry condition of the finger, in which a structure for spraying water, a skin lotion, a moisturizer, or the like on the finger surface or a surface of the transparent cover 313 that makes contact with the finger surface is arranged in the vicinity of the sensor 211 of the acquisition unit 21 or the like, whereby when the dry condition score calculated in Step S41 is lower than a predetermined value, the water, skin lotion, moisturizer, or the like can be automatically or manually sprayed on the finger surface or the surface of the transparent cover 313, or the user is encouraged to spray the water, skin lotion, moisturizer, or the like. In addition, the transparent cover 313 itself may be made of a material capable of adjusting moisture, a material capable of saturating moisture, or the like so as to move from a wet portion to a dry portion, or may have a structure capable of adjusting moisture by providing a groove or the like on the surface of the transparent cover 313, and any method can be employed.

Note that the other configurations, operations, and effects may be similar to those of the embodiments described above, and detailed description thereof will be omitted here.

### 5. Hardware configuration

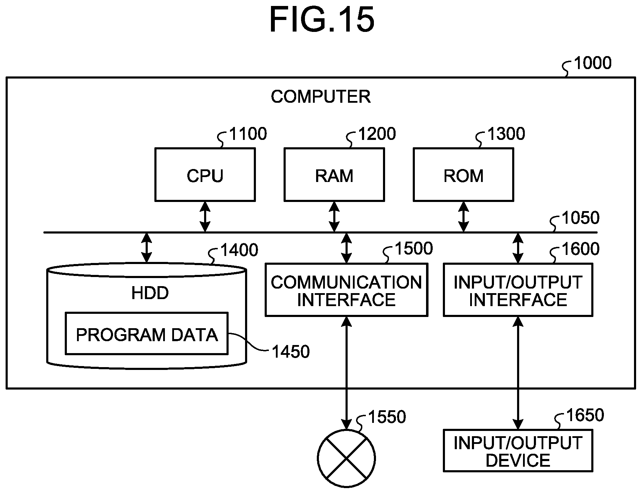

The information processing apparatus 10 according to the embodiments and modifications described above, can be implemented by, for example, a computer 1000 having a configuration as illustrated in FIG. 15. FIG. 15 is a hardware configuration diagram illustrating an example of the computer 1000 implementing the functions of the information processing apparatus 10. The computer 1000 includes a CPU 1100, a RAM 1200, a read only memory (ROM) 1300, a hard disk drive (HDD) 1400, a communication interface 1500, and an input/output interface 1600. The respective units of the computer 1000 are connected by a bus 1050.

The CPU 1100 is operated on the basis of programs stored in the ROM 1300 or the HDD 1400 and controls the respective units. For example, the CPU 1100 deploys a program stored in the ROM 1300 or the HDD 1400 to the RAM 1200, and performs processing corresponding to each of various programs.

The ROM 1300 stores a boot program, such as a basic input output system (BIOS), executed by the CPU 1100 upon boosting the computer 1000, a program depending on hardware of the computer 1000, and the like.

The HDD 1400 is a computer-readable recording medium that non-transitorily records the programs performed by the CPU 1100, data used by the programs, and the like. Specifically, the HDD 1400 is a recording medium that records a program for performing each operation according to the present disclosure that is an example of program data 1450.

The communication interface 1500 is an interface for connecting the computer 1000 to an external network 1550 (e.g., the Internet). For example, the CPU 1100 receives data from another device or transmits data generated by the CPU 1100 to another device, via the communication interface 1500.

The input/output interface 1600 has a configuration including an I/F unit 18 described above, and is an interface that connects between an input/output device 1650 and the computer 1000. For example, the CPU 1100 receives data from an input device such as a keyboard or mouse, via the input/output interface 1600. In addition, the CPU 1100 transmits data to an output device such as a display, speaker, or printer, via the input/output interface 1600. Furthermore, the input/output interface 1600 may function as a media interface that reads a program or the like recorded on a predetermined recording medium. The medium includes, for example, an optical recording medium such as a digital versatile disc (DVD) or phase change rewritable disk (PD), a magneto-optical recording medium such as a magneto-optical disk (MO), a tape medium, a magnetic recording medium, a semiconductor memory, or the like.

For example, when the computer 1000 functions as the information processing apparatus 10 according to the embodiments described above, the CPU 1100 of the computer 1000 executes the programs loaded on the RAM 1200 to implement the functions of the information processing apparatus 10. Furthermore, the HDD 1400 stores the programs according to the present disclosure, and the like. Note that the CPU 1100 executes the program data 1450 read from the HDD 1400, but in another example, the CPU 1100 may acquire these programs from another apparatus via the external network 1550.

Furthermore, the computer 1000 is merely an example and is not limited to a disk top type, notebook type, laptop type, or tablet type personal computer, and the technology of the present disclosure can also be applied to various information processing devices such as a wristband type wearable device, ring type wearable device, headphone, smartphone, stationary type electronic device, in-vehicle device for an automobile or the like, head mount display (HMD), and augmented reality (AR) glasses.

Note that the embodiments of the present technology are not limited to the embodiments described above, and various modifications and alterations can be made without departing from the spirit and scope of the present technology. Moreover, the component elements of different embodiments and modifications may be suitably combined with each other.

Furthermore, the present technology can have the following configurations.
(1) An information processing apparatus including:
   an acquisition unit that acquires image information including biometric information of a target;
   a condition recognition unit that determines a condition of the target based on the image information;
   a projection unit that projects light on the target based on a result of the determination by the condition recognition unit; and
   an authentication unit that performs authentication based on the image information of the target acquired by the acquisition unit in a state where the light is projected.
(2) The information processing apparatus according to (1), wherein
   the condition recognition unit identifies an area in the image information where the biometric information is lost, and
   the projection unit projects the light for complementing the biometric information of the area where the biometric information is lost, on the target.
(3) The information processing apparatus according to (2), wherein
   the projection unit projects, as the light, an ideal image including ideal biometric information about the target, on the target.
(4) The information processing apparatus according to (2) or (3), wherein
   the projection unit selectively projects the light, based on the area where the biometric information is lost.
(5) The information processing apparatus according to any one of (2) to (4), wherein
   the projection unit projects, as the light, complementary information for complementing the biometric information of the area where the biometric information generated by machine learning based on the image information acquired by the acquisition unit is lost, on the target.
(6) The information processing apparatus according to any one of (1) to (5), further including
   a position detection unit that detects a position of the target,
   wherein the projection unit projects the light on the target, based on the position of the target detected by the position detection unit.
(7) The information processing apparatus according to (6), wherein
   the position detection unit estimates, based on the image information of the target acquired by the acquisition unit and ideal image information including ideal biometric information about the target, a positional relationship between the image information and the ideal image information.
(8) The information processing apparatus according to any one of (1) to (7), wherein
   the projection unit controls at least one of a projection angle of the light, a focus of a lens included in the acquisition unit, and a wavelength of the light.
(9) The information processing apparatus according to any one of (1) to (8), wherein
   the condition recognition unit determines the condition of the target, based on the image information to acquire condition information indicating the condition of the target.
(10) The information processing apparatus according to (9), wherein
   the condition information includes at least one of information indicating a specific condition of the target and information obtained by scoring a degree of dryness.
(11) The information processing apparatus according to any one of (1) to (10), further including
   a presentation unit that presents information for reducing an area where the biometric information is lost.
(12) The information processing apparatus according to (11), wherein
   the presentation unit presents information for moving the target to an area having fewer areas where the biometric information is lost.
(13) The information processing apparatus according to (11) or (12), wherein
   the target is a skin of a user, and
   the information for reducing the area where the biometric information is lost includes recommendation information that is recommended to the user to improve the condition of the target.
(14) The information processing apparatus according to (13), wherein
   the recommendation information includes recommended product information that indicates a product recommended to the user, and
   the recommended product information includes at least one of condition information indicating the condition of the target and date, time, temperature, and humidity upon identification of the product, in addition to information for identifying the product identified based on the condition of the target.
(15) The information processing apparatus according to (13) or (14), wherein
   the presentation unit presents a change between before use and after use of the product in chronological order, based on the recommended product information.
(16) The information processing apparatus according to (14) or (15), wherein
   the presentation unit confirms user's intention to purchase the product based on the recommended product information, based on the authentication by the authentication unit.
(17) The information processing apparatus according to any one of (1) to (16), wherein
   the biometric information includes at least one of fingerprint information, palm print information, vein information, iris information, and eye vein information.
(18) The information processing apparatus according to any one of (9) to (12), wherein
   the target is a finger of a user, and
   the condition of the target includes a dry condition on a finger surface.
(19) An information processing method including:
   acquiring image information including biometric information of a target;
   determining a condition of the target based on the image information;
   projecting light on the target based on a result of the determination; and
   performing authentication based on the image information of the target acquired by the acquisition unit in a state where the light is projected.
(20) A program causing
   a computer to function as:
   an acquisition unit that acquires image information including biometric information of a target;
   a condition recognition unit that determines a condition of the target based on the image information;
   a projection unit that projects light on the target based on a result of the determination by the condition recognition unit; and
   an authentication unit that performs authentication based on the image information of the target acquired by the acquisition unit in a state where the light is projected.

### Reference Signs List

- 10: INFORMATION PROCESSING APPARATUS
- 11: CALCULATION UNIT
- 12: CONTROL UNIT
- 13: STORAGE UNIT
- 14: COMMUNICATION UNIT
- 21: ACQUISITION UNIT
- 22: CONDITION RECOGNITION UNIT
- 23: POSITION DETECTION UNIT
- 31: PROJECTION UNIT
- 32: AUTHENTICATION UNIT
- 33: PRESENTATION UNIT
- 211: SENSOR
- 310: INFORMATION PROCESSING APPARATUS
- 311: LIGHT SOURCE
- 312: PROJECTION OPTICAL SYSTEM
- 313: TRANSPARENT COVER
- 314: LIGHT SOURCE CHANGING UNIT
- 331: ARITHMETIC UNIT
- 332: OUTPUT UNIT
- 900: NETWORK
- 901: FINGER
- 910: INFORMATION PROCESSING APPARATUS
- 1000: COMPUTER
- 1100: CPU
- 1200: RAM
- 1300: ROM
- 1400: HDD
- 1450: PROGRAM DATA
- 1500: COMMUNICATION INTERFACE
- 1550: EXTERNAL NETWORK
- 1600: INPUT/OUTPUT INTERFACE
- 1650: INPUT/OUTPUT DEVICE
- C1: OPTICAL AXIS
- C2: OPTICAL AXIS

## Claims

1. An information processing apparatus including:
an acquisition unit that acquires image information including biometric information of a target;
a condition recognition unit that determines a condition of the target based on the image information;
a projection unit that projects light on the target based on a result of the determination by the condition recognition unit; and
an authentication unit that performs authentication based on the image information of the target acquired by the acquisition unit in a state where the light is projected.

2. The information processing apparatus according to claim 1, wherein
the condition recognition unit identifies an area in the image information where the biometric information is lost, and
the projection unit projects the light for complementing the biometric information of the area where the biometric information is lost, on the target.

3. The information processing apparatus according to claim 2, wherein
the projection unit projects, as the light, an ideal image including ideal biometric information about the target, on the target.

4. The information processing apparatus according to claim 2, wherein
the projection unit selectively projects the light, based on the area where the biometric information is lost.

5. The information processing apparatus according to claim 2, wherein
the projection unit projects, as the light, complementary information for complementing the biometric information of the area where the biometric information generated by machine learning based on the image information acquired by the acquisition unit is lost, on the target.

6. The information processing apparatus according to claim 1, further including
a position detection unit that detects a position of the target,
wherein the projection unit projects the light on the target, based on the position of the target detected by the position detection unit.

7. The information processing apparatus according to claim 6, wherein
the position detection unit estimates, based on the image information of the target acquired by the acquisition unit and ideal image information including ideal biometric information about the target, a positional relationship between the image information and the ideal image information.

8. The information processing apparatus according to claim 2, wherein
the projection unit controls at least one of a projection angle of the light, a focus of a lens included in the acquisition unit, and a wavelength of the light.

9. The information processing apparatus according to claim 1, wherein
the condition recognition unit determines the condition of the target, based on the image information to acquire condition information indicating the condition of the target.

10. The information processing apparatus according to claim 9, wherein
the condition information includes at least one of information indicating a specific condition of the target and information obtained by scoring a degree of dryness.

11. The information processing apparatus according to claim 1, further including
a presentation unit that presents information for reducing an area where the biometric information is lost.

12. The information processing apparatus according to claim 11, wherein
the presentation unit presents information for moving the target to an area having fewer areas where the biometric information is lost.

13. The information processing apparatus according to claim 11, wherein
the target is a skin of a user, and
the information for reducing the area where the biometric information is lost includes recommendation information that is recommended to the user to improve the condition of the target.

14. The information processing apparatus according to claim 13, wherein
the recommendation information includes recommended product information that indicates a product recommended to the user, and
the recommended product information includes at least one of condition information indicating the condition of the target and date, time, temperature, and humidity upon identification of the product, in addition to information for identifying the product identified based on the condition of the target.

15. The information processing apparatus according to claim 14, wherein
the presentation unit presents a change between before use and after use of the product in chronological order, based on the recommended product information.

16. The information processing apparatus according to claim 14, wherein
the presentation unit confirms user's intention to purchase the product based on the recommended product information, based on the authentication by the authentication unit.

17. The information processing apparatus according to claim 1, wherein
the biometric information includes at least one of fingerprint information, palm print information, vein information, iris information, and eye vein information.

18. The information processing apparatus according to claim 11, wherein
the target is a finger of a user, and
the condition of the target includes a dry condition on a finger surface.

19. An information processing method including:
acquiring image information including biometric information of a target;
determining a condition of the target based on the image information;
projecting light on the target based on a result of the determination; and
performing authentication based on the image information of the target acquired by the acquisition unit in a state where the light is projected.

20. A program causing
a computer to function as:
an acquisition unit that acquires image information including biometric information of a target;
a condition recognition unit that determines a condition of the target based on the image information;
a projection unit that projects light on the target based on a result of the determination by the condition recognition unit; and
an authentication unit that performs authentication based on the image information of the target acquired by the acquisition unit in a state where the light is projected.
